# EUROPEAN PATENT APPLICATION

(11) **EP 3 690 033 A1**
(43) Date of publication of application: **05.08.2020**
(21) Application number: 18860318.7
(22) Date of filing: 27.09.2018
(51) Int. Cl.: C12N 5/10, A61K 35/17, A61P 35/00

(54) **ENGINEERED IMMUNE CELL CAPABLE OF INDUCING SECRETION OF ANTI-CD47 ANTIBODY**

(30) Priority: 27.09.2017 CN 201710891841
(71) Applicant: Gracell Biotechnologies (Shanghai) Co., Ltd., Shanghai 200233 (CN)
(72) Inventor: ZHANG, Yongliang, Shanghai 200233 (CN); LIU, Liping, Shanghai 200233 (CN); CAO, Wei, Shanghai 200233 (CN); MA, Ling, Shanghai 200233 (CN); MA, Anyun, Shanghai 200233 (CN); HE, Jiaping, Shanghai 200233 (CN); SHEN, Lianjun, Shanghai 200233 (CN); WANG, Xinxin, Shanghai 200233 (CN)
(74) Representative: Cabinet Laurent & Charras
(86) International application number: PCT/CN2018/108022
(87) International publication number: WO 2019/062817

(57) **Abstract**

Provided are an immune cell capable of inducing the secretion of an anti-CD47 antibody when a CAR and/or an exogenous TCR is activated, a use thereof, and a preparation comprising the immune cell. Also provided are a preparation method of the immune cell and a kit for the preparation method.

## Description

### Technical field

The invention belongs to the field of tumor immune cell therapy, and particularly relates to an engineered immune cell capable of inducing the secretion of an anti-CD47 antibody.

### Background

Cellular immunotherapy is an emerging and highly effective tumor treatment model, and is a new type of immunotherapy for cancer. It is a method for *in vitro* culture and amplification of immune cells collected from a patient using biotechnology and biological agents, which are then transfused back to the patient to stimulate and enhance the body's immune function, thereby achieving the purpose of treating tumors.

In recent years, as "living drugs", chimeric antigen receptor genetically modified T (CAR-T) cells have achieved exciting results in the treatment of hematological tumors, and have become a new development direction for tumor treatment. The design of CARs has gone through the following process. The first generation CAR has only one intracellular signal component, CD3ζ or FcyRI molecule. Because there is only one activation domain in the cell, it can only cause transient T cell proliferation and less cytokine secretion, and does not provide long-term T cell proliferation signals and sustained antitumor effects *in vivo.* Therefore, it has not achieved very good clinical efficacy. The second generation CAR is introduced with a costimulatory molecule based on the original structure, such as CD28, 4-1BB, OX40, and ICOS. Compared with the first generation CAR, the function has been greatly improved, and the sustainability of CAR-T cells and the ability to kill tumor cells are further enhanced. Based on the second generation CAR, some new immune stimulatory molecules such as CD27 and CD134 were linked in tandom to develop the third and fourth generation CARs. Currently, the second-generation CAR is most commonly used in clinical trials of blood tumors.

CAR-T cells have shown unprecedented efficacy in the treatment of hematological malignancies. For example, the complete remission (CR) can reach 90% in the treatment of advanced relapsed refractory acute lymphoblastic leukemia (ALL), and the CR is over 50% for chronic lymphocytic leukemia (CLL) and some B-cell lymphomas. Although CAR-T has great potential in the treatment of leukemia and lymphoma, it is not effective in treating many solid tumors and some hematomas. At present, CAR-T cell therapy still has problems such as off-target effects, toxic and side effects, short duration *in-vivo,* and high recurrence rate in the treatment of hematological tumors. The safety and effectiveness of CAR-T cells in the treatment of solid tumors have been proved, but the efficacy needs to be improved.

CD47 is a potential target for the treatment of tumors. At present, researches mainly focus on the use of antibodies targeting CD47 for tumor treatment. However, since CD47 is commonly expressed in normal tissues, systemic infusion of antibodies will bring many on-target off-tumor toxic side effects, such as anemia and neurotoxicity. Therefore, antibodies targeting CD47 are rarely used to treat CD47-expressing tumors.

In summary, there is still a need for further research in the field to develop an engineered immune cell that can treat tumors more effectively with better specificity and less side effects.

### Summary of the invention

The objective of the present invention is to provide an engineered immune cell (such as CAR-T cell) which can treat tumor more effectively, with good specificity and less side effect.

Another objective of the present invention is to provide an engineered immune cell (such as CAR-T cell) capable of inducing the secretion of anti-CD47 antibodies, as well as a preparation method and application thereof.

According to a first aspect of the present invention, it provides an engineered immune cell which is a T cell or an NK cell with following characteristics:
(a) the immune cell expresses a chimeric antigen receptor CAR or an exogenous TCR, wherein the CAR targets a marker of tumor cells, and the exogenous TCR targets a marker of tumor cells; and
(b) when the CAR is activated and/or the exogenous TCR is activated, the immune cell induces the secretion of anti-CD47 antibodies.

In another preferred embodiment, the engineered immune cell is selected from the group consisting of:
(i) chimeric antigen receptor T cell (CAR-T cell);
(ii) chimeric antigen receptor NK cell (CAR-NK cell); or
(iii) exogenous T cell receptor (TCR) T cell (TCR-T cell).

In another preferred embodiment, it provides a chimeric antigen receptor T cell (CAR-T cell) with following characteristics:
(a) the cell expresses a chimeric antigen receptor CAR, and the CAR targets a marker of tumor cells; and
(b) when the CAR is activated, the CAR-T cell induce the secretion of anti-CD47 antibodies.

In another preferred embodiment, the anti-CD47 antibody is selected from the group consisting of an antibody from an animal species, a chimeric antibody, a humanized antibody, and a combination thereof.

In another preferred embodiment, the anti-CD47 antibody is a partially or fully humanized antibody.

In another preferred embodiment, the anti-CD47 antibody is in a form of single-chain or double-chain.

In another preferred example, the anti-CD47 antibody includes a plurality of (2, 3, or 4) single-chain antibodies in tandom.

In another preferred example, in the plurality of (2, 3, or 4) single-chain antibodies in tandom, a linker peptide La is located between two adjacent single-chain antibodies.

In another preferred embodiment, the linker peptide La is 5-25 amino acids, preferably 10-20 amino acids in length.

In another preferred example, the linker peptide is flexible.

In another preferred example, the "activation" refers to the binding of the CAR or exogenous TCR to a marker of tumor cells.

In another preferred example, the "tumor marker" refers to a tumor-specific antigen.

In another preferred example, the chimeric antigen receptor CAR or exogenous TCR is located on the cell membrane of the engineered immune cell.

In another preferred example, the chimeric antigen receptor CAR is located on the cell membrane of the CAR-T cell.

In another preferred embodiment, the structure of the CAR is shown in formula I:

L1-scFv-H1-TM-C-CD3ζ (I)

wherein,
L1 is none or a signal peptide sequence;
scFv is an antigen binding domain;
H1 is none or a hinge region;
TM is a transmembrane domain;
C is a co-stimulatory signaling molecule;
CD3ζ is a cytoplasmic signaling sequence derived from CD3ζ;
the "-" is a linker peptide or a peptide bond;

In another preferred embodiment, the L1 is the signal peptide of a protein selected from the group consisting of CD8, GM-CSF, CD4, CD137, and a combination thereof. Preferably, the sequence of L is as shown in positions 1-22 of SEQ ID NO: 1.

In another preferred embodiment, the scFv is an antibody single-chain variable region sequence targeting a tumor antigen.

In another preferred embodiment, the scFv is an antibody single-chain variable region sequence targeting an antigen selected from the group consisting of CD19, CD20, CD22, CD123, CD47, CD138, CD33, CD30, mesothelin (MSLN), EGFR, GPC3, BCMA, ErbB2, NKG2D ligands, LMP1, EpCAM, VEGFR-1, Lewis-Y, ROR1, Claudin 18.2, and a combination thereof.

In another preferred embodiment, the scFv is an antibody single-chain variable region sequence targeting CD 19.

In another preferred embodiment, the scFv is FMC63, and the sequence is as shown in positions 23-270 of SEQ ID NO: 1.

In another preferred embodiment, the scFv is an antibody single-chain variable region sequence targeting MSLN.

In another preferred embodiment, the scFv is P4, and the sequence is as shown in positions 22-279 of SEQ ID NO: 5.

In another preferred embodiment, the H is the hinge region of a protein selected from the group consisting of CD8, CD28, CD137, and a combination thereof.

In another preferred embodiment, the H1 is a hinge region derived from CD28, and preferably the sequence of H1 is as shown in positions 271-309 of SEQ ID NO: 1.

In another preferred embodiment, the TM is the transmembrane region of a protein selected from the group consisting of CD28, CD3ε, CD45, CD4, CD5, CD8, CD9, CD16, CD22, CD33, CD37, CD64, CD80, CD86, CD134, CD137, CD154, and a combination thereof.

In another preferred embodiment, the TM is a transmembrane region derived from CD28, and preferably the sequence of TM is as shown in positions 310-336 of SEQ ID NO: 1.

In another preferred embodiment, the C is the co-stimulatory signaling molecule of a protein selected from the group consisting of OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, and a combination thereof.

In another preferred example, C is a co-stimulatory signaling molecule derived from CD28, and preferably the sequence of C is as shown in positions 337-377 of SEQ ID NO: 1.

In another preferred example, the sequence of CD3ζ is as shown in positions 378-489 of SEQ ID NO: 1.

In another preferred embodiment, the structure of the CAR targeting CD19 is L-FMC63-CD28-CD3ζ.

In another preferred embodiment, the structure of the CAR targeting MSLN is L-P4-CD28-CD3ζ.

In another preferred embodiment, the sequence of the CAR is as shown in SEQ ID NO: 1 or 5.

In another preferred example, the anti-CD47 antibody is an anti-CD47 scFv.

In another preferred embodiment, the structure of the anti-CD47 scFv is shown in formula II as below:

L2-VH-X-VL-H2-G (II)

wherein,
L2 is none or a signal peptide sequence;
VH is a heavy chain variable region of anti-CD47 antibody;
X is none or a linker peptide;
VL is a light chain variable region of anti-CD47 antibody;
H2 is none or a hinge region of an immunoglobulin;
G is none or an Fc fragment.

In another preferred embodiment, the L2 is the signal peptide of a protein selected from the group consisting of CD8, GM-CSF, CD4, CD137, and a combination thereof. Preferably, the sequence of L2 is as shown in positions 1-21 of SEQ ID NO: 2.

In another preferred embodiment, the sequence of VH is as shown in positions 22-139 of SEQ ID NO: 2.

In another preferred embodiment, the sequence of VL is as shown in positions 155-261 of SEQ ID NO: 2.

In another preferred embodiment, the X is 2-50 amino acids, preferably 3-30 amino acids in length.

In another preferred embodiment, the X is (G4S)_{N}, and N is a positive integer from 1 to 8.

In another preferred embodiment, the X is (G4S)₃.

In another preferred embodiment, the sequence of X is as shown in positions 140-154 of SEQ ID NO: 2.

In another preferred embodiment, the H2 is the hinge region of a protein selected from the group consisting of IgG1, IgG2, IgG3, IgG4, and a combination thereof.

In another preferred embodiment, the H2 is selected from IgG1.

In another preferred embodiment, the amino acid sequence of the anti-CD47 scFv is as shown in SEQ ID NO: 2, SEQ ID NO: 4 or SEQ ID NO: 6.

In a second aspect of the invention, it provides a method for preparing the engineered immune cell of the first aspect of the invention, comprising the following steps:
(A) providing an immune cell to be modified; and
(B) modifying the immune cell to express a CAR or an exogenous TCR, wherein when the CAR is activated and/or the exogenous TCR is activated, the immune cell induces the secretion of anti-CD47 antibodies, thereby obtaining the engineered immune cell of the first aspect of the invention.

In another preferred embodiment, the step (B) comprises (B1) transferring a first expression cassette expressing the CAR or exogenous TCR into the immune cell; and (B2) transferring a second expression cassette which can induce the secretion of anti-CD47 antibodies into the immune cell; wherein step (B1) may be performed before, after, at the same time, or alternately with step (B2).

In another preferred embodiment, it provides a method for preparing the CAR-T cell of the first aspect of the invention, comprising the following steps:
(A) providing a T cell to be modified; and
(B) modifying the T cell to express the CAR and secrete anti-CD47 antibodies when the CAR is activated, thereby obtaining the CAR-T cell of the first aspect of the invention.

In another preferred embodiment, the step (B) comprises (B1) transferring a first expression cassette expressing the CAR into the T cell; and (B2) transferring a second expression cassette which can induce the secretion of anti-CD47 antibodies into the T cell; wherein step (B1) may be performed before, after, at the same time, or alternately with step (B2).

In another preferred embodiment, the first expression cassette comprises a nucleic acid sequence encoding the chimeric antigen receptor (CAR).

In another preferred embodiment, the second expression cassette has a structure of formula III from 5'-3':

Z1-Z2 (III)

wherein,
each "-" is independently a bond or a nucleotide linking sequence;
Z1 is an inducible promoter;
Z2 is a nucleic acid sequence encoding an anti-CD47 antibody.

In another preferred embodiment, the Z1 is an NFAT inducible promoter, preferably an NFAT-IL2 mixed promoter.

In another preferred embodiment, Z1 contains 4, 5, or 6 NFAT binding domains and an IL-2 promoter (preferably a fragment of IL-2 minimal promoter) from 5' to 3 '.

In another preferred embodiment, the sequence of Z1 is as shown in positions 1-297 of SEQ ID NO: 3.

In another preferred embodiment, the sequence of Z2 is as shown in positions 361-1080 of SEQ ID NO: 3.

In another preferred embodiment, the sequence of the second expression cassette is as shown in SEQ ID NO: 3.

In another preferred embodiment, when the T cell to be modified in step (A) expresses a certain CAR, the step (B) comprises (B2) transferring the second expression cassette into the T cell.

In another preferred embodiment, the transcription directions of the first expression cassette and the second expression cassette are the same (→→), opposing (→ ←), or opposite (← →).

In another preferred embodiment, the first expression cassette and the second expression cassette are located on the same or different vectors.

In another preferred embodiment, the first expression cassette and the second expression cassette are located on the same vector.

In another preferred embodiment, the vector is a virus vector.

In another preferred embodiment, the vector is selected from the group consisting of DNA, RNA, plasmid, lentiviral vector, adenoviral vector, retroviral vector, transposon, other gene transfer systems, and a combination thereof.

In another preferred embodiment, the vector is a FUW lentiviral vector.

In a third aspect of the invention, it provides a preparation comprising the engineered immune cell of the first aspect of the invention, and a pharmaceutically acceptable carrier, diluent or excipient.

In another preferred embodiment, it provides a preparation comprising the CAR-T cell of the first aspect of the invention, and a pharmaceutically acceptable carrier, diluent or excipient.

In another preferred embodiment, the preparation is a liquid preparation.

In another preferred embodiment, the formulation of the preparation comprises injection.

In another preferred embodiment, the concentration of the CAR-T cells in the preparation is 1 × 10³-1 × 10⁸ cells/ml, preferably 1 × 10⁴⁻1 × 10⁷ cells/ml.

In a fourth aspect of the invention, it provides a use of the engineered immune cell of the first aspect of the invention for the preparation of a medicament or a preparation for preventing and/or treating cancer or tumor.

In another preferred embodiment, it provides a use of the CAR-T cell of the first aspect of the invention for the preparation of a medicament or a preparation for preventing and/or treating cancer or tumor.

In another preferred embodiment, the tumor is selected from the group consisting of a hematological tumor, a solid tumor, and a combination thereof.

In another preferred embodiment, the hematological tumor is selected from the group consisting of acute myeloid leukemia (AML), multiple myeloma (MM), chronic lymphocytic leukemia (CLL), acute lymphoblastic leukemia (ALL), diffuse large B cell lymphoma (DLBCL), and a combination thereof.

In another preferred embodiment, the solid tumor is selected from the group consisting of gastric cancer, peritoneal metastasis of gastric cancer, liver cancer, leukemia, renal cancer, lung cancer, small intestine cancer, bone cancer, prostate cancer, colorectal cancer, breast cancer, large intestine cancer, cervical cancer, ovarian cancer, lymphoma, nasopharyngeal carcinoma, adrenal tumor, bladder tumor, non-small cell lung cancer (NSCLC), glioma, and a combination thereof.

In another preferred embodiment, the tumor is a tumor with high CD47 expression.

In another preferred embodiment, the tumor is selected from the group consisting of B-cell lymphoma, non-Hodgkin's lymphoma, ovarian cancer, and a combination thereof.

In a fifth aspect of the invention, it provides a kit for preparing the engineered immune cell of the first aspect of the invention, wherein the kit comprises a container and following components located in the container:
(1) a first nucleic acid sequence comprising a first expression cassette for expressing the CAR or exogenous TCR; and
(2) a second nucleic acid sequence comprising a second expression cassette for inducing secretion of anti-CD47 antibodies.

In another preferred embodiment, it provides a kit for preparing the CAR-T cell according to the first aspect of the invention, wherein the kit comprises a container and following components located in the container:
(1) a first nucleic acid sequence comprising a first expression cassette for expressing the CAR; and
(2) a second nucleic acid sequence comprising a second expression cassette for inducing secretion of anti-CD47 antibodies.

In another preferred embodiment, the first and the second nucleic acid sequences are independent or connected.

In another preferred embodiment, the first and the second nucleic acid sequences are located in the same or different containers.

In another preferred embodiment, the first and the second nucleic acid sequences are located on the same or different vectors.

In another preferred embodiment, the first and the second nucleic acid sequences are located on the same vector.

In another preferred embodiment, the vector is a viral vector, and preferably the viral vector comprises the first and the second nucleic acid sequences in a tandem form.

It is to be understood that the various technical features of the present invention mentioned above and the various technical features specifically described hereinafter (as in the Examples) may be combined with each other within the scope of the present invention to constitute a new or preferred technical solution, which needs not be described one by one, due to space limitations.

### Description of Drawings

Figure 1 shows a schematic structure of the CAR in Example 1, wherein A shows a structure of a CAR targeting CD19, and B shows a structure of a CAR targeting MSLN. In the figure, L is a signal peptide.
Figure 2 shows a schematic structure of the expression cassette capable of inducing secretion of aCD47scFv in Example 1. Wherein, IL-2 TATA is an IL-2 mini promoter and HA is a tag.
Figure 3 shows a schematic structure of the expression cassette capable of inducing secretion of aCD47scFv-Fc in Example 1.
Figure 4 shows the effective killing of target cells by MSLN CAR-T. Wherein, A shows the expression of MSLN CAR; B shows the target cell NCI-H226 that highly expresses MSLN antigen; C shows the RTCA killing experiment result that MSLN CAR-T effectively kills target cell of NCI-H226; D shows that a large amount of IFN-γ is secreted by MSLN CAR-T cell which is activated by an antigen.
Figure 5 shows detection of the expression of anti-CD47scFV single chain antibody/anti-CD47scFV-FC antibody in supernatant. A shows the schematic of gene expression frame of the vector; B shows the expression of anti-CD47scFV single chain antibody in 293T cells; C shows the expression of anti-CD47scFV-FC antibody in Jurkat T cells. EF-1 α is a constitutive promoter.

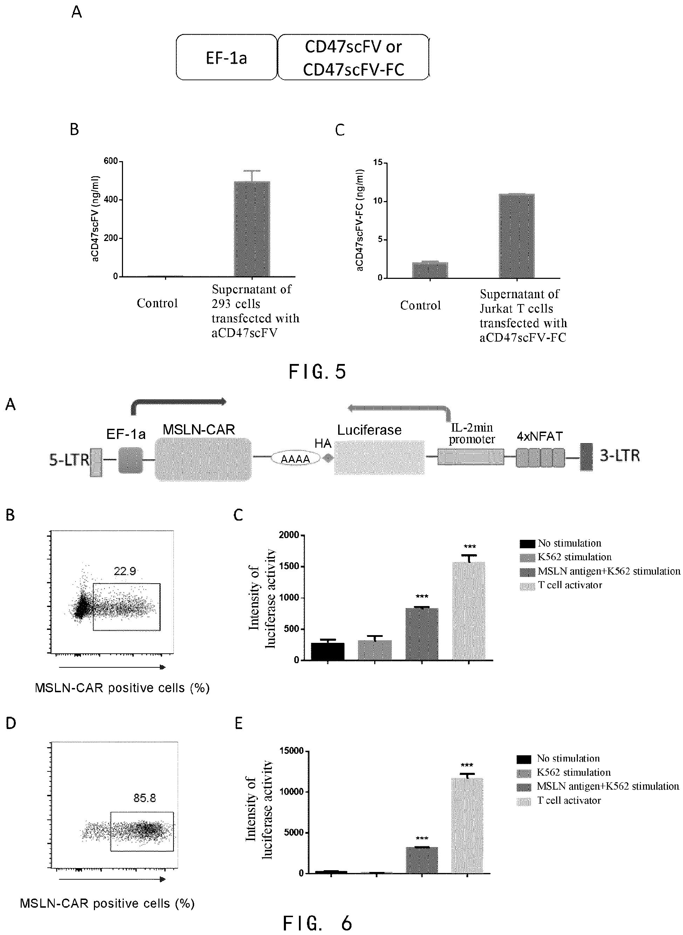

Figure 6 shows that MSLN CAR-T binds antigen and induces downstream gene expression. A shows a schematic structure of CAR gene induced for expression; B shows that Jurkat T cell electrotransformed the expression vector of A stably expresses MSLN CAR and binds to K562 cells that overexpress MSLN antigen; C shows the inducible expression of secreted luciferase; D shows that T cells isolated from peripheral blood were infected with virus packaged with expression vector, then the T cells stably expressed MSLN CAR and binded to K562 cells that overexpress MSLN antigen; E shows the inducible expression of secreted luciferase.
Figure 7 shows a schematic of the gene expression frame of iCD47scFV secretion induced by activation of MSLN CAR-T antigen.
Figure 8 shows that anti-CD47scFV single-chain antibody can promote the phagocytosis of tumor cells by bone marrow-derived macrophages, wherein A and B show the phagocytosis of Nalm6 by bone marrow-derived macrophages, and the effect of aCD47scFV supernatant compared with the control group was analyzed with flow cytometry and statistics (** P <0.01); C and D show the phagocytosis of K562 by bone marrow-derived macrophages, and the effect of aCD47scFV supernatant compared with the control group was analyzed with flow cytometry and statistics (* P <0.05).
Figure 9 shows that anti-CD47scFV single chain antibody synergistically promotes the killing of tumor K562 by macrophage and MSLN CAR-T.

### Embodiments for Carrying Out the Present Invention

The present invention takes CAR-T cells as an example to representatively describe the engineered immune cells of the present invention in detail. The engineered immune cells of the present invention are not limited to the CAR-T cells described in the context, and have the same or similar technical features and beneficial effects as the CAR-T cells described in the context. Specifically, when the immune cells express the chimeric antigen receptor CAR, NK cells are equivalent to T cells (or T cells can be replaced with NK cells); when immune cells are T cells, TCR is equivalent to CAR (or CAR can be replaced by TCR).

After extensive and intensive research and screening, the present inventors combined CAR with an anti-CD47 antibody for the first time, and unexpectedly discovered a CAR-T cell that can induce the secretion of anti-CD47 antibodies. Experiments show that the present invention can use anti-CD47 antibodies to kill CD47 positive tumor cells without causing side effects. The CAR-T cell of the present invention initiates the transcription and translation of anti-CD47 antibody only when the CAR is activated, so as to achieve the function of specifically secretion only in the tumor microenvironment. The CAR-T cell does not secrete the CD47 antibodies in normal tissues or blood, which can avoid systemic on-target off-tumor toxicity and side effects without disturbing normal tissues *in vivo.* The CAR-T cell of the present invention can induce the secretion of anti-CD47 antibodies, relieve the inhibition of macrophages by CD47-positive tumor cells, and insteadly promote macrophages to attack tumor cells. Moreover, the anti-CD47 antibody cooperate with the CAR to better exert the anti-tumor effect. The killing effect of tumor cells is significantly enhanced. The CAR-T cell can simultaneously kill tumor cells expressing CAR-targeted antigens and CD47-positive tumor cells, preventing immune escape of tumor cells, off target and relapse. On this basis, the present invention has been completed.

### Terms

To make the disclosure easier to understand, some terms are firstly defined. As used in this application, unless expressly stated otherwise herein, each of the following terms shall have the meanings given below. Other definitions are set forth throughout the application.

The term "about" may refer to a value or composition within an acceptable error range for a particular value or composition as determined by those skilled in the art, which will depend in part on how the value or composition is measured or determined.

The term "administering" refers to the physical introduction of a product of the invention into a subject using any one of various methods and delivery systems known to those skilled in the art, including intravenous, intramuscular, subcutaneous, intraperitoneal, spinal or other parenteral administration, such as by injection or infusion.

### Antibody

As used herein, the term "antibody" (Ab) may include, but is not limited to, an immunoglobulin that specifically binds an antigen and contains at least two heavy (H) chains and two light (L) chains linked by disulfide bonds, or an antigen binding parts thereof. Each H chain contains a heavy chain variable region (abbreviated herein as VH) and a heavy chain constant region. The heavy chain constant region contains three constant domains, CH1, CH2, and CH3. Each light chain contains a light chain variable region (abbreviated herein as VL) and a light chain constant region. The light chain constant region contains a constant domain CL. The VH and VL regions can be further subdivided into hypervariable regions called complementarity determining regions (CDR), which are interspersed within more conservative regions called framework regions (FR). Each VH and VL contains three CDRs and four FRs, which are arranged from amino terminal to carboxy terminal in the following order: FR1, CDR1, FR2, CDR2, FR3, CDR3, and FR4. The variable regions of the heavy and light chains contain a binding domain that interacts with an antigen.

### Antigen binding domain

As used herein, the "antigen binding domain" and "single-chain antibody fragment" refer to a Fab fragment, a Fab' fragment, an F(ab')₂ fragment, or a single Fv fragment that has antigen-binding activity. The Fv antibody contains the heavy chain variable region and the light chain variable region of the antibody, but has no constant region. The Fv antibody has the smallest antibody fragment with all antigen-binding sites. Generally, Fv antibodies also include a polypeptide linker between the VH and VL domains, and can form the structure required for antigen binding. The antigen binding domain is usually a scFv (single-chain variable fragment). The single-chain antibody is preferably an amino acid chain sequence encoded by a nucleotide chain. As a preferred mode of the invention, the scFv comprises an antibody that specifically recognizes an antigen highly expressed by tumors, preferably a single-chain antibody or Fv antibody.

In the present invention, the anti-CD47 antibody is a scFv antibody that targets CD47. In the present invention, "anti-CD47 scFv", "CD47 scFV" and "anti-CD47 antibody" are used interchangeably, and are all scFvs targeting CD47, including aCD47 scFV, aCD47 scFv-FC, and the like. Preferably, the sequence of the anti-CD47 antibody is as shown in SEQ ID NO: 2, SEQ ID NO: 4 or SEQ ID NO: 6.

In another preferred embodiment, the amino acid sequence of aCD47 scFv is as shown in SEQ ID NO: 2.

MALPVTALLLPLALLLHAARPEVQLVESGGDLVKPGGSLKLSCAASGFTFSGYGM
SWVRQTPDKRLEWVATITSGGTYTYYPDSVKGRFTISRDNAKNTLYLQIDSLKSEDTAI
YFCARSLAGNAMDYWGQGTSVTVSSGGGGSGGGGSGGGGSDIVMTQSPATLSVTPGD
RVSLSCRASQTISDYLHWYQQKSHESPRLLIKFASQSISGIPSRFSGSGSGSDFTLSINSVE
PEDVGVYYCQNGHGFPRTFGGGTKLEIK (SEQ ID NO: 2)

In another preferred embodiment, the amino acid sequence of aCD47 scFv-FC is as shown in SEQ ID NO: 4.

MALPVTALLLPLALLLHAARPEVQLVESGGDLVKPGGSLKLSCAASGFTFSGYGM
SWVRQTPDKRLEWVATITSGGTYTYYPDSVKGRFTISRDNAKNTLYLQIDSLKSEDTAI
YFCARSLAGNAMDYWGQGTSVTVSSGGGGSGGGGSGGGGSDIVMTQSPATLSVTPGD
RVSLSCRASQTISDYLHWYQQKSHESPRLLIKFASQSISGIPSRFSGSGSGSDFTLSINSVE
PEDVGVYYCQNGHGFPRTFGGGTKLEIKEPKSCDKTHTCPPCPAPELLGGPSVFLFPPKP
KDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 4)

In another preferred embodiment, the anti-CD47 antibody is a humanized antibody and the amino acid sequence thereof is as shown in SEQ ID NO: 6.

MGVKVLFALICIAVAEAEVQLVESGGGLVQPGGSLRLSCAASGFTFSGYGMSWVR
QAPGKGLEWVATITSGGTYTYYPDSVKGRFTISRDNAKNSLYLQMNSLRAEDTAVYY
CARSLAGNAMDYWGQGTLVTVSSGGGGSGGGGSGGGGSEIVLTQSPATLSLSPGERA

TLSCRASQSISDYLHWYQQKPGQAPRLLIYFASQRATGIPARFSGSGSGTDFTLTISSLEP
EDFAVYYCQQGHGFPRTFGGGTKVEIK (SEQ ID NO: 6)

### Chimeric antigen receptor (CAR)

As used herein, the chimeric immune antigen receptor (CAR) includes an extracellular domain, an optional hinge region, a transmembrane domain, and an intracellular domain. The extracellular domain comprises an optional signal peptide and a target-specific binding element (also known as an antigen binding domain). The intracellular domain includes a co-stimulatory molecule and a ζ chain. When the CAR is expressed in T cells, the extracellular region can recognize a specific antigen, and then transduce this signal through the intracellular domain, causing the cell activation and proliferation, cytolytic toxicity, and secretion of cytokines, such as IL-2 and IFN-γ and so on. This affects tumor cells, causing them to not grow, be prompted to die, or be affected in other ways, and leading to a reduction or elimination of the patient's tumor burden. The antigen binding domain is preferably fused to the intracellular domain from one or more of the co-stimulatory molecule and the ζ chain. Preferably, the antigen binding domain is fused with an intracellular domain of a combination of a CD28 signaling domain and a CD3ζ signaling domain.

In one embodiment, the CAR of the present invention targets CD19 and can specifically bind to CD19. In another preferred embodiment, the structure of the present CAR is L-FMC63-CD28-CD3ζ. Preferably, the sequence of the present CAR is as shown in SEQ ID NO: 1.

In one embodiment, the CAR of the present invention targets MSLN and can specifically bind to MSLN.

In another preferred example, the structure of the CAR targeting MSLN is L-P4-CD28-CD3ζ, and preferably, the amino acid sequence of the CAR is as shown in SEQ ID NO: 5.

### Exogenous T cell antigen receptor (TCR)

As used herein, exogenous T cell antigen receptor (TCR) is α and β chains of TCR cloned from tumor-reactive T cells by gene transfer technology. The exogenous TCR is transferred into T cells with lentivirus or retrovirus as a vector by means of genetic engineering.

Exogenous TCR-modified T cells can specifically recognize and kill tumor cells. By optimizing the affinity of TCR and tumor-specific antigens, the affinity between T cells and tumors can be improved and the anti-tumor effect can be improved.

### Chimeric antigen receptor T cell (CAR-T cell)

As used herein, the terms "CAR-T cell", "CAR-T", "CAR-T cell of the invention" all refer to the CAR-T cell of the first aspect of the invention. The CAR-T cell of the present invention can be used to treat tumors with high expression of CD47, such as B-cell lymphoma, non-Hodgkin's lymphoma, ovarian cancer, and the like.

CAR-T cells have the following advantages over other T-cell-based treatments: (1) the role of CAR-T cells is not restricted by MHC; (2) since many tumor cells express same tumor antigen, once the construction of a CAR gene targeting a certain tumor antigen is completed, it can be widely used; (3) CAR can use both tumor protein antigens and glycolipid non-protein antigens, thereby expanding the target range of tumor antigens; (4) the use of patient's autologous cells reduces the risk of rejection reaction; (5) CAR-T cells have the immune memory function and can survive *in vivo* for a long time.

### Chimeric antigen receptor NK cell (CAR-NK cell)

As used herein, the terms "CAR-NK cell", "CAR-NK", "CAR-NK cell of the invention" all refer to the CAR-NK cell of the first aspect of the invention. The CAR-NK cell of the present invention can be used to treat tumors with high expression of CD47, such as B-cell lymphoma, non-Hodgkin's lymphoma, ovarian cancer, and the like.

Natural killer (NK) cells are a major class of immune effector cells that protect the body from viral infection and invasion of tumor cells through non-antigen-specific pathways. Engineered (genetically modified) NK cells may obtain new functions, including the ability to specifically recognize tumor antigens and enhanced anti-tumor cytotoxicity.

Compared with autologous CAR-T cells, CAR-NK cells also have the following advantages, for example: (1) they directly kill tumor cells by releasing perforin and granzyme, but have no killing effect on normal cells of the body; (2) they release small amount of cytokines, which reduces the risk of cytokine storm; (3) they are easy to expand *in vitro,* which can develop into "off-the-shelf products. In addition to this, it is similar to CAR-T cell therapy.

### CD47

CD47 is a member of the Ig superfamily. It consists of an extracellular amino-terminal Ig-like variable domain (ligand binding region), five hydrophobic transmembrane fragments, and a carboxy-terminal intracellular tail region. CD47 is widely expressed on the surface of different tissue cells, such as hematopoietic cells (red blood cells, lymphocytes, platelets, etc.), non-hematopoietic cells (placental, liver, brain cells, etc.) and tumor cells. CD47 is highly expressed in leukemia stem cells, such as AML, blastic phase of chronic myeloid leukemia (CML-BP), and T-cell acute lymphoblastic leukemia. CD47 expression is found in a variety of tumor tissues, including multiple myeloma, bladder cancer, rectal cancer, melanoma and so on. Although CD47 is expressed in normal tissues, the expression level is significantly lower than that in tumor tissues.

CD47 is highly expressed in many tumor cells, and tumor cells highly express CD47 to avoid macrophage phagocytosis. CD47 acts as a self-signal, and tumor cells evade the phagocytosis of macrophages through the expression of anti-phagocytosis signals. In lymphocytes, CD47 binds to its specific ligand SIRPα to form a CD47-SIRPα signal complex, which can send anti-phagocytosis signals and inhibit phagocytosis of phagocytic cells, causing insight holes of immune system, and promoting tumor development.

The expression level of CD47 in peripheral blood and germinal center-like B cells in patients with B-cell lymphoma is significantly higher than that in normal B cells. At the same time, the study also found that CD47 is expressed in non-Hodgkin's lymphoma (NHL) of different pathophysiological types, such as diffuse large B-cell lymphoma (DLBCL), follicular cell lymphoma (FL), and marginal zone lymphoma (MZL), mantle cell lymphoma (FCL), etc.

CD47 is a potential target for the treatment of tumors. At present, researches mainly focus on the use of antibodies targeting CD47 for tumor treatment. CD47 antibody treatment exerts tumor killing effect through DC cells and CD8+ T cells. DC cells synergize with phagocytic molecules through CD47 antibodies to phagocytose tumor cells and present tumor-associated antigens to CD8+ T cells, thereby exerting the specific killing effect of CD8+ T cells on tumors. However, since CD47 is commonly expressed in normal tissues, systemic infusion of antibodies will bring many on-target off-tumor toxic side effects, such as anemia and neurotoxicity. Therefore, the inventors have developed a chimeric antigen receptor T cell that is induced to express secretory CD47 scFV only when it is specifically activated by tumor antigens. Especially for solid tumors, the CAR-T cell can directly deliver CD47 antibodies to the tumor microenvironment and relieve the inhibitory effect of tumor cells on macrophages, thereby exerting the phagocytosis of macrophages and achieving an anti-tumor effect.

### Nuclear factor of activated T cells (NFAT)

Activated T cell nuclear factor (NFAT) is a family of transcription factors, which plays an important role in inducing gene transcription in immune responses. In resting cells, NFAT exists in the cytoplasm and is in an inactive phosphorylated state called NF-ATp, which has a low affinity for DNA. When tumor antigens are specifically recognized by CAR-T cells, T cells are specifically activated and mediate Ca²⁺ influx, thereby activating the calcineurin activity and inducing the dephosphorylation of NFAT. The dephosphorylation activates NFAT and allows it to enter the nucleus, to bind to the promoter of related genes, and to induce gene expression.

In the present invention, the inventor designed an expression vector. The promoter region contains 4, 5, or 6 regions capable of binding to NFAT, followed by a smallest fragment of IL-2 promoter, and meanwhile a CD47 antibody sequence is placed after the promoter region. When the CAR-T cell is in a resting state, it does not secrete anti-CD47 antibodies. Only after the cell is activated by tumor-specific antigens, NFAT will be dephosphorylated and activated, and then NFAT will enter the nucleus and regulate the secretion of CD47 antibodies. The specific secretion of anti-CD47 antibodies in the tumor microenvironment is achieved, so as to remove the inhibitory effect of tumor cells on macrophages, exhibit the anti-tumor activity, and avoid systemic off-target toxicity.

### Real-time label-free cell analysis

Using Real Time Cellular Analysis (RTCA), the dynamic detection of immune cell killing and the evaluation of optimal ratio of effective cells to target cells can be achieved without any labeling. The RTCA technology is based on the principle of electrical impedance, and detects the biological appearance of adherent cell. For suspended cells added to the well, they do not cause electrical impedance changes because they do not contact or weakly contact the electrode on the bottom of detection plate.

### Expression cassette

As used herein, "expression cassette" or "expression cassette of the invention" includes the first expression cassette and the second expression cassette. The expression cassette of the invention is described in the fifth aspect of the present invention. The first expression cassette comprises a nucleic acid sequence encoding the CAR. The second expression cassette has a structure of formula A from 5' to 3'. When the CAR is activated by a tumor-specific antigen, the second expression cassette expresses the anti-CD47 antibody. When the CAR-T cell of the present invention is in a resting state and the CAR does not bind to the specific antigen, the second expression cassette does not express the anti-CD47 antibody.

In one embodiment, the first expression cassette and the second expression cassette each further includes a promoter and/or a terminator, wherein the promoter of the second expression cassette is an inducible promoter, preferably an NFAT inducible promoter, more preferably, a fragment containing 4, 5, or 6 NFAT-binding domains and a IL-2 minimal promoter.

### Vector

The present invention also provides a vector containing the expression cassette of the present invention. Vectors derived from retroviruses such as the lentivirus are suitable tools to achieve long-term gene transfer since they allow long-term, stable integration of a transgene and its propagation in daughter cells. Lentiviral vectors have the advantage over vectors derived from onco-retroviruses such as murine leukemia viruses in that they can transduce non-proliferating cells, such as hepatocytes. They also have the advantage of low immunogenicity.

In brief summary, the expression cassette or nucleic acid sequence of the invention is typically and operably linked to a promoter, and incorporated into an expression vector. The vectors can be suitable for replication and integration in eukaryotes. Typical cloning vectors contain transcription and translation terminators, initiation sequences, and promoters useful for regulation of the expression of the desired nucleic acid sequence.

The expression constructs of the present invention may also be used for nucleic acid immune and gene therapy, using standard gene delivery protocols. Methods for gene delivery are known in the art. See, e.g., U.S, Pat. Nos. 5,399,346, 5,580,859, 5,589,466, incorporated by reference herein in their entireties.

The expression cassette or the nucleotide sequence can be cloned into a number of types of vectors. For example, the expression cassette or the nucleotide sequence can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al, (2001 , Molecular Cloning: A Laboratory Manual, Cold Spring Harbor Laboratory, New York), and in other virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S, Pat. No. 6,326, 193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-la (EF- 1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the invention should not be limited to the use of constitutive promoters, inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionein promoter, a glucocorticoid promoter, a progesterone promoter, and a tetracycline promoter.

The expression vector to be introduced into a ceil can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, e.g., mammalian (such as human T cell), bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. For example, see U.S. Pat, Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

In a preferred embodiment of the invention, the vector is a lentiviral vector.

### Preparation

The invention provides a preparation comprising the CAR-T cell according to the first aspect of the invention, and a pharmaceutically acceptable carrier, diluent or excipient. In one embodiment, the preparation is a liquid preparation. Preferably, the preparation is an injection. Preferably, the concentration of the CAR-T cells in the preparation is 1 × 10³⁻1 × 10⁸ cells/ml, more preferably 1 × 10⁴- 1 × 10⁷ cells/ml.

In one embodiment, the preparation may comprises buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. The preparation of the invention is preferably formulated for intravenous administration.

### Therapeutic application

The invention comprises therapeutic applications using cells (e.g., T cells) transduced with a lentiviral vector (LV) comprising the expression cassette of the invention. The transduced T cells can target the tumor cell marker and specifically secrete anti-CD47 antibodies. The T cells synergistically activate macrophages, and meanwhile cause immune response of T cells and macrophages, thereby significantly increasing the killing efficiency against tumor cells.

Thus, the present invention also provides a method for stimulating a T cell-mediated immune response to a target cell population or tissue in a mammal comprising the step of administering to the mammal a CAR-T cell of the invention.

In one embodiment, the present invention comprises a class of cell therapies, wherein autologous T cells from a patient (or heterologous donor) are isolated, activated and genetically modified to generate CAR-T cells, and then injected into the same patient. The probability of graft versus host disease in the way is extremely low, and antigens are recognized by T cells in a non-MHC-restricted manner. In addition, one kind of CAR-T can treat all cancers that express the antigen. Unlike antibody therapies, CAR-T cells are able to replicate *in vivo* resulting in long-term persistence that can lead to sustained tumor control

In one embodiment, the CAR-T cells of the invention can undergo robust in vivo T cell expansion and can persist for an extended amount of time. In addition, the CAR mediated immune response may be part of an adoptive immunotherapy approach in which CAR-modified T cells induce an immune response specific to the antigen binding moiety in the CAR. For example, an anti-CD19 CAR-T cell elicits an immune response specifically against cells expressing CD19. An anti-MSLN CAR-T cell elicits an immune response specifically against cells expressing MSLN.

Cancers that may be treated include tumors that are unvascularized or largely unvascularized, and tumors that are vascularized. Cancers may include non-solid tumors (such as hematological tumors, for example, leukemias and lymphomas) or solid tumors. Types of cancers to be treated with the CARs of the invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblasts, promyeiocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors, such as sarcomas and carcinomas, include fibrosarcoma, myxosarcoma, liposarcoma, mesothelioma, malignant lymphoma, pancreatic cancer and ovarian cancer.

The CAR-T cells of the invention may also serve as a type of vaccine for *ex vivo* immunization and/or *in vivo* therapy in a mammal. Preferably, the mammal is a human.

With respect to *ex vivo* immunization, at least one of the following occurs *in vitro* prior to administering the cell into a mammal: i) expanding the cells, ii) introducing the expression cassette of the invention to the cells, and/or iii) cryopreservation of the cells.

*Ex vivo* procedures are well known in the art and are discussed more fully as below. Briefly, cells are isolated from a mammal (preferably a human) and genetically modified (i.e., transduced or transfected *in vitro*) with a vector comprising the expression cassette of the invention. The CAR-T cell of the invention can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

In addition to using a cell-based vaccine in terms of *ex vivo* immunization, the present invention also provides compositions and methods for *in vivo* immunization to elicit an immune response directed against an antigen in a patient.

Generally, the activated and expanded cells as described herein can be used for treating and preventing disease occurring in an individual without an immune response. Therefore, the present invention provides methods for treating cancers comprising administering to a subject in need thereof, a therapeutically effective amount of the CAR-modified T cells of the invention.

The CAR-T cells of the present invention may be administered either alone, or as a pharmaceutical composition in combination with diluents and/or with other components such as IL-2, IL-17 or other cytokines or cell populations. Briefly, pharmaceutical compositions of the present invention may comprise a target cell population as described herein, in combination with one or more pharmaceutically or physiologically acceptable carriers, diluents or excipients.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount", "an anti-tumor effective amount", "an tumor-inhibiting effective amount", or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 10⁴ to 10⁹ cells/kg body weight, preferably 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al" New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous (*i.v.*) injection, or intraperitoneally. In one embodiment, the T cell compositions of the present invention are administered to a patient by intradermal or subcutaneous injection. In another embodiment, the T cell compositions of the present invention are preferably administered by *i.v.* injection. The compositions of T cells may be injected directly into a tumor, lymph node, or site of infection.

In certain embodiments of the present invention, cells activated and expanded using the methods described herein, or other methods known in the art where T cells are expanded to therapeutic levels, are administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. In further embodiments, the T cells of the invention may be used in combination with chemotherapy, radiation, immunosuppressive agents, such as cyclosporin, azathioprine, methotrexate, mycophenolate, and FK506, antibodies, or other immunotherapeutic agents. In a further embodiment, the cell compositions of the present invention are administered to a patient in conjunction with (e.g., before, simultaneously or following) bone marrow transplantation, or the use of chemotherapy agents such as, fludarabine, external-beam radiation therapy (XRT), cyclophosphamide. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for human administration can be performed according to art-accepted practices. In general, 1x10⁵ to 1x10¹⁰ of the modified T cells of the invention can be applied to patients by means of, for example, intravenous reinfusion each treatment or each course of treatment.

### The main advantages of the invention

(1) The present invention can use anti-CD47 antibodies to kill CD47 positive tumor cells without causing side effects. The CAR-T cell of the present invention only initiates the transcription and translation of an anti-CD47 antibody when the CAR is activated, so as to achieve the function of specifically secretion only in the tumor microenvironment. The CAR-T cell does not secrete CD47 antibodies in normal tissues or blood, which can avoid systemic on-target off-tumor toxicity and side effects without disturbing normal tissues *in vivo.* The CAR-T cell is safe and has little toxic and side effects.
(2) The CAR-T cell of the present invention can induce the secretion of an anti-CD47 antibodies, relieve the inhibition of macrophages by CD47-positive tumor cells, and instead promote macrophages to attack tumor cells. Moreover, the anti-CD47 antibody cooperate with the CAR to better exert the anti-tumor effect. The killing effect of tumor cells is significantly enhanced. The CAR-T cell can simultaneously kill tumor cells expressing CAR-targeted antigens and CD47-positive tumor cells, preventing immune escape of tumor cells, off target and relapse.
(3) As for solid tumors, the CAR-T cell can directly deliver anti-CD47 antibodies to the tumor microenvironment and relieve the inhibitory effect of tumor cells on macrophages, thereby exerting the phagocytosis of macrophages and achieving an anti-tumor effect.
(4) The anti-CD47 antibody of the present invention has an Fc fragment, which can bind to the Fc receptor on the surface of NK cells to activate NK cells and enable NK cells to exert the killing effect, so as to achieve a better anti-tumor effect. The Fc fragment can also improve the stability of the scFV of the invention. The anti-CD47 antibody of the present invention also comprises a humanized CD47 antibody, which is less immunogenic and has less toxic and side effects.

The present invention will be further illustrated below with reference to the specific examples. It is to be understood that these examples are for illustrative purposes only and are not intended to limit the scope of the invention. For the experimental methods in the following examples the specific conditions of which are not specifically indicated, they are performed under routine conditions, e.g., those described by Sambrook. et al., in Molecule Clone: A Laboratory Manual, New York: Cold Spring Harbor Laboratory Press, 1989, or as instructed by the manufacturers, unless otherwise specified. Unless indicated otherwise, parts and percentage are weight parts and weight percentage.

### Materials and Methods

### 1. Peripheral blood mononuclear cells PBMC were isolated from donor blood and T cells were expanded.

Monocytes were isolated from cord blood. Histopaque-1077 (Sigma-Aldrich) was used for density gradient centrifugation and T cells were enriched (using EasySep human T cell enrichment kit, Stemcell Technologies). T cells were activated, cultured and expanded using anti-CD3/anti-CD28 conjugated magnetic beads. X-vivo15 (containing 5% FBS, 2mM L-glutamine, ImM sodium pyruvate, 300IU/ml rhIL2) was used as the culture medium. All cells were cultured in an incubator at 37 °C, 5% CO₂.

### 2. Culture of cells

Jurkat T cells (human T lymphocyte leukemia cell line, ATCC® TIB-152)
Nalm6 cells (human acute lymphocytic leukemia cell line, ATCC® CRL-3273)
Raji cells (Burkitt's lymphoma cells, ATCC-CCL86);
Raji-ffluc cell line (obtained after screening of Raji cells infected with lentivirus expressing firefly luciferase);
K562-ffluc cells (human erythroleukemia cell line, ATCC-CCL243);
293T cells (human kidney epithelial cell line, ATCC-CRL3216);
K562 cells and 293T cells expressing CD19 were obtained by screening after infection with CD19-expressing lentiviral vectors.

K562 cells and 293T cells expressing MSLN were obtained by screening after infection with MSLN-expressing lentiviral vectors.

Jurkat T, Nalm6, Raji cells, Raji-ffluc, K562, K562 cells expressing CD19, and K562 cells expressing MSLN were cultured using RPMI1640 medium. 293T cells, 293T cells expressing CD19, and 293T cells expressing MSLN were cultured using DMEM medium. All media were supplemented with 10% (v/v) fetal calf serum and 100 U/ml of avidin and streptomycin, 2 mM L-glutamine, and 1 mM sodium pyruvate. All cells were cultured in a constant temperature incubator at 37 °C, 5% CO₂.

### Example 1 Design and transduction of CAR structure and iCD47scFv structure

### 1.1 Structure design of the CAR targeting CD 19 (referred to as CD 19CAR)

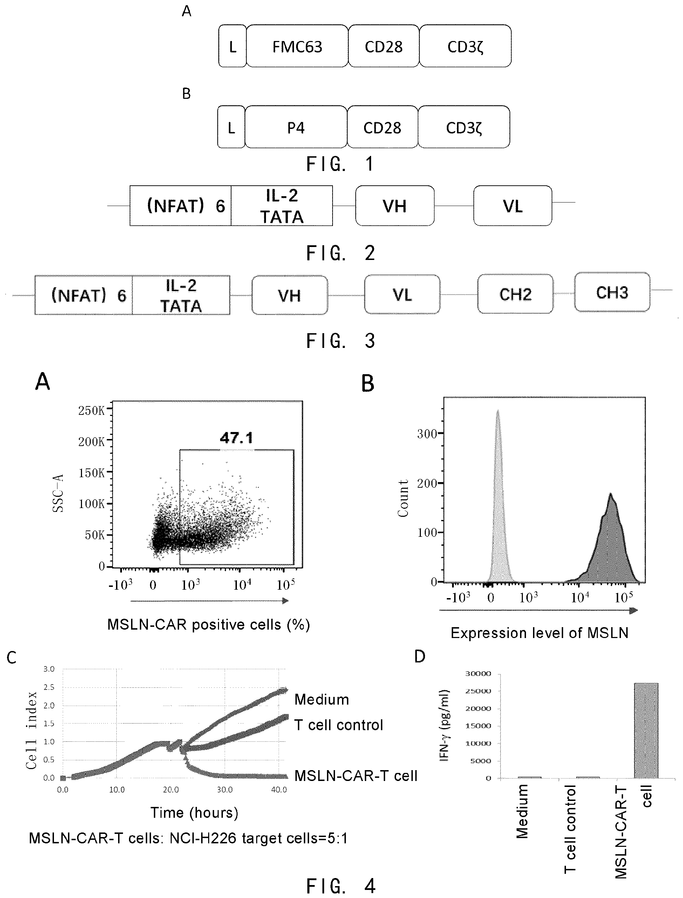

As for the structure of CD19CAR, a second generation CD19 CAR is used, which comprises an scFv from FMC63, a hinge and transmembrane region from CD28, and the intracellular region is CD28 and CD3ζ. The schematic structure is shown in Figure 1A, and the amino acid sequence is as shown in SEQ ID NO: 1.

MLLLVTSLLLCELPHPAFLLIPDIQMTQTTSSLSASLGDRVTISCRASQDISKYLNW
YQQKPDGTVKLLIYHTSRLHSGVPSRFSGSGSGTDYSLTISNLEQEDIATYFCQQGNTLP
YTFGGGTKLEITGSTSGSGKPGSGEGSTKGEVKLQESGPGLVAPSQSLSVTCTVSGVSLP
DYGVSWIRQPPRKGLEWLGVIWGSETTYYNSALKSRLTIIKDNSKSQVFLKMNSLQTD
DTAIYYCAKHYYYGGSYAMDYWGQGTSVTVSSAAAIEVMYPPPYLDNEKSNGTIIHV
KGKHLCPSPLFPGPSKPFWVLVVVGGVLACYSLLVTVAFIIFWVRSKRSRLLHSDYMN
MTPRRPGPTRKHYQPYAPPRDFAAYRSRVKFSRSADAPAYQQGQNQLYNELNLGRRE
EYDVLDKRRGRDPEMGGKPRRKNPQEGLYNELQKDKMAEAYSEIGMKGERRRGKGH
DGLYQGLSTATKDTYDALHMQALPPR (SEQ ID NO: 1)

The CAR-T cell targeting CD19 stably expresses CAR gene. CAR has an artificially designed amino acid sequence, comprising a signal peptide, a scFv, a hinge region, a transmembrane region, and an intracellular signal region connected in sequence. Wherein, the vector expressing the CAR gene can be DNA, RNA, plasmid, lentiviral vector, adenoviral vector, retroviral vector, transposon, or other gene transfer systems.

The CD19 CAR gene was cloned into the FUW lentiviral vector framework and placed downstream of the EF1α promoter to form Fuw-EF1α-CD19CAR. The three plasmids Fuw-EF1α-CD19CAR, pMD2.G and psPAX2 (addgene) were transferred into 293T using Lipofectamine3000 to prepare a lentiviral expression vector. The virus supernatants were collected at 48h and 72h, and concentrated by ultracentrifugation (Merck Millipore). The concentrated virus was then used to infect T cells.

### 1.2 Structure design of the CAR targeting MSLN (referred to as MSLN-CAR)

As for the structure of MSLNCAR, a second generation MSLN CAR is used, which comprises an scFv from P4, a hinge and transmembrane region from CD28, and the intracellular region is CD28 and CD3ζ. The schematic structure is shown in Figure 1B, and the amino acid sequence is as shown in SEQ ID NO: 5, wherein the scFv, i.e. the antigen recognition sequence of the CAR-T targeting MSLN is indicated by underscore.

MALPVTALLLPLALLLHAARPQVQLQQSGPGLVTPSQTLSLTCAISGDSVSSNSAT
WNWIRQSPSRGLEWLGRTYYRSKWYNDYAVSVKSRMSINPDTSKNQFSLQLNSVTPE
DTAVYYCARGMMTYYYGMDVWGQGTTVTVSSGILGSGGGGSGGGGSGGGGSQPVL
TQSSSLSASPGASASLTCTLRSGINVGPYRIYWYQQKPGSPPQYLLNYKSDSDKQQGSG
VPSRFSGSKDASANAGVLLISGLRSEDEADYYCMIWHSSAAVFGGGTQLTVLTTTPAPR
PPTPAPTIASQPLSLRPEACRPAAGGAVHTRGLDFACDFWVLVVVGGVLACYSLLVTV
AFIIFWVRSKRSRLLHSDYMNMTPRRPGPTRKHYQPYAPPRDFAAYRSRVKFSRSADA
PAYQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPR (SEQ ID NO: 5)

### 1.3 Structural design of the inducible CD47scFv expression vector (referred to as iCD47scFv)

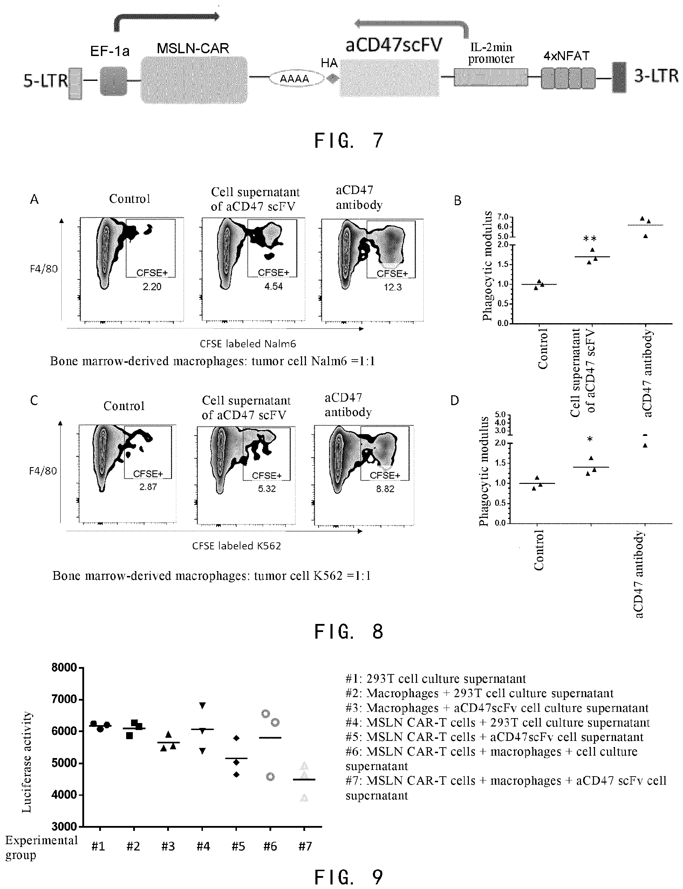

The present invention designed an expression cassette that can induce the secretion of an anti-CD47scFv (the signal peptide is selected from CD8). The schematic structure is shown in Figure 2 (aCD47 scFv) or Figure 3 (aCD47 scFv-FC). The amino acid sequences of aCD47 scFv and aCD47 scFv-FC are shown in SEQ ID NO: 2 and SEQ ID NO: 4, respectively.

The nucleotide sequence of the expression cassette NFAT-IL-2-aCD47 scFv that can induce the secretion of aCD47scFv (the signal peptide is selected from CD8) is as shown in SEQ ID NO: 3. By replacing the aCD47scFv coding sequence (positions 361-1080) in the sequence as shown in SEQ ID NO: 3 with the aCD47scFv-FC coding sequence, the expression cassette that can induce the secretion of aCD47scFv-FC (the signal peptide is selected from CD8) is obtained.

1 ggaggaaaaa ctgtttcata cagaaggcgt ggaggaaaaa ctgtttcata cagaaggcgt
61 ggaggaaaaa ctgtttcata cagaaggcgt ggaggaaaaa ctgtttcata cagaaggcgt
121 ggaggaaaaa ctgtttcata cagaaggcgt ggaggaaaaa ctgtttcata cagaaggcgt
181 tttgacaccc ccataatatt tttccagaat taacagtata aattgcatct cttgttcaag
241 agttccctat cactctcttt aatcactact cacagtaacc tcaactcctg ccacaatatg
301 gccttaccag tgaccgcctt gctcctgccg ctggccttgc tgctccacgc cgccaggccg
361 gaggtgcagc tggtggagtc tgggggagac ttagtgaagc ctggagggtc cctgaaactc
421 tcctgtgcag cctctggatt cactttcagt ggctatggca tgtcttgggt tcgccagact
481 ccagacaaga ggctggagtg ggtcgcaacc attactagtg gtggtactta cacctactat
541 ccagacagtg tgaaggggcg attcaccatc tccagagaca atgccaagaa caccctgtac
601 ctgcaaatag acagtctgaa gtctgaggat acagccatat atttctgtgc aagatccctc

661 gcgggaaatg ctatggacta ctggggtcaa gggaccagcg tcaccgtctc ctcaggtggc
721 ggtggttctg gtggcggtgg ttctggtggc ggtggttctg atattgtgat gactcagtct
781 ccagccaccc tgtctgtgac tccaggagat agagtctctc tttcctgcag ggccagccag
841 actattagcg actacttaca ctggtatcaa caaaaatcac atgagtctcc aaggcttctc
901 atcaaatttg cttcccaatc catttctgga atcccctcca ggttcagtgg cagtggatca
961 ggctcagatt tcactctcag tatcaacagt gtggaacctg aagatgttgg agtgtattac
1021 tgtcaaaatg gtcacggctt tcctcggacg ttcggtggag ggaccaagct ggaaataaaa (SEQ ID NO: 3)

The expression cassette of the anti-CD47 antibody fragment which is placed downstream of the NFAT-IL-2 promoter was cloned into the FUW lentiviral vector framework containing the CD19 CAR or MSLN CAR gene to form Fuw-EF1α-CD19CAR- NFAT-IL-2-CD47scFv or Fuw-EF1α-MSLNCAR-NFAT-IL-2-CD47scFv. It was transferred into 293T cell together with pMD2.G and psPAX2 (Addgene) using Lipofectamine3000 to prepare a lentiviral expression vector. The virus supernatants were collected at 48h and 72h, and concentrated by ultracentrifugation (Merck Millipore). The concentrated virus was then used to infect T cells.

### Example 2 Preparation of CAR-T cells

The isolated and purified primary T cells were activated for 3 days, and then the cells were infected with a lentiviral expression vector comprising MSLN-CAR and MSLN-CAR-iCD47scFv. The cells were transferred to cell culture flasks, and cultured in a constant temperature incubator at 37°C, 5% CO₂. The CAR positive rate of T cells was detected with MSLN (Thermo Fisher Scientific) on the 3rd and 7th day after infection. Half of the medium was changed every 2-3 days. The CAR-T cells obtained after the culture were MSLN CAR-T cells and iCD47scFv-MSLN CAR-T cells, respectively. Wherein, the experimental results of MSLN CAR-T cells are shown in Figure 4A. MSLN CAR-T cells can express MSLN CAR well.

Similarly, iCD47scFv-MSLN CAR-T cells can also express MSLN CAR well.

CD19 CAR-T cells and iCD47scFv-CD19CAR-T cells were prepared according to the same experimental methods described above. It was found that CD19 CAR-T cells and iCD47scFv-CD19CAR-T cells can express CD19 CAR well. CD19 CAR-T cells and iCD47scFv-CD19CAR-T cells were successfully prepared.

### Example 3 Killing ability of CAR-T cells in vitro

### 3.1 Killing ability of MSLN CAR-T cells in vitro

Using Real Time Cellular Analysis (RTCA), the dynamic detection of immune cell killing and the evaluation of optimal ratio of effector cells to target cells can be achieved without any labeling. The RTCA technology is based on the principle of electrical impedance, and detects the biological appearance of adherent cell. For suspended cells added to the well, they do not cause electrical impedance changes because they do not contact or weakly contact the electrode on the bottom of detection plate. Therefore, the monolayer cancer cell killing mediated by CAR-T cells can be directly monitored quantitatively using RTCA technology. CAR-T cells prepared in Example 2 were used as effector cells, and MSLN-positive mesothelioma cells NCI-H226 (Fig. 4B) were used as target cells. The cells were co-cultured in a ratio of E: T = 5:1. The instant and long-term killing ability of CAR-T cells to tumors was analyzed and obtained by continuous detection of the killing of tumor cells by CAR-T cells.

The results showed that MSLN-positive CAR-T cells (MSLN-CAR) could rapidly and effectively kill MSLN-positive tumor cells compared with T cells that were not transfected with CAR structure (NT) in the control group (Figure 4C).

Similarly, iCD47scFv-MSLN CAR-T cells could also rapidly and effectively kill MSLN-positive tumor cells.

### 3.2 Killing ability of CD 19 CAR-T cells in vitro

The experimental method is the same as 3.1 above, wherein MSLN CAR-T cells were replaced with CD19 CAR-T cells, and mesothelioma cells were replaced with CD19-positive tumor cells. The results showed that CD19 CAR-T cells could quickly and effectively kill CD19-positive tumor cells compared with T cells that were not transfected with CAR structure (NT) in the control group.

### Example 4 Cytokine release assay of CAR-T cells

The co-cultured supernatant (co-cultured for 42 hours) of MSLN CAR-T cells and tumor cells NCI-H226 cells obtained in Example 3 was collected and centrifuged. Then the cytokine release level of IFN-γ was detected using an Elisa kit (Biolegend).

The results showed that the release level of IFN-γ in MSLN-CAR cell group was significantly higher than that in the control group (Figure 4D).

Similarly, the release level of IFN-γ of iCD47scFv-MSLN CAR-T cells was significantly higher than that of the control group.

According to the above method, the effector cells were replaced with CD19 CAR-T cells, and mesothelioma cells were replaced with CD19-positive tumor cells. The results showed that the release level of IFN-γ in CD19 CAR-T cell group was significantly higher than that in the control group.

### Example 5 Construction and expression of aCD47 scFV vector

The expression cassette (Figure 5A) comprising the nucleotide sequence encoding the constitutive promoter EF-1α and aCD47 scFv (SEQ ID NO: 2) or aCD47scFv-FC (SEQ ID NO: 4) was cloned into p-fuw-EF-1□lentiviral expression vector. According to the method of 1.3 in Example 1, three plasmids pMD2.G and psPAX2 (Addgene) were transferred into 293T cells using Lipofectamine3000 to prepare lentiviral expression vectors. The virus supernatants were collected at 48h and 72h, and concentrated by ultracentrifugation (Merck Millipore). The concentrated virus was then used to infect 293T cells and cell lines stably expressing aCD47 scFv or aCD47 scFv-FC were obtained.

293FT cells were infected with the virus. Then the expression and content of aCD47 scFv (SEQ ID NO: 2) in the supernatant of the stably transfected cells were detected by ELISA method. It was found that the aCD47 scFV expression vector could express well in eukaryotic cells 293T, and can reach a high level of 494 ng/ml (Figure 5B).

Similarly, the vector expressing aCD47scFv-FC (SEQ ID NO: 4) was introduced to Jurkat T cells by a Lonza electrotransformation apparatus. The cell supernatant was collected after 18 hours. The secretion of aCD47-scFv-FC was also detected in the cell supernatant by ELISA method, and the expression abundance was 10.5 ng/ml (Figure 5C).

### Example 6 System detection of T cell inducible expression

The present invention hopes that the CAR-T cell can directly deliver anti-CD47 antibodies to the tumor microenvironment, and relieve the inhibitory effect of tumor cells on macrophages, thereby exerting the phagocytosis of macrophages. For this reason, in the design of the present invention (as shown in Figure 6A), the CAR-T cells recognizing MSLN get to the tumor site, then bind to the MSLN tumor antigen, activate and up-regulate the downstream NFAT transcription factor, and start the antibody secretion program.

### 6.1 A secreted luciferase is used as a reporter gene

The Jurkat T cells were transfected with the lentiviral vector of Figure 6A by electrotransfection, as shown in Figure 6B. The transfected Jurkat T cells expressed MSLN CAR. After 4 hours of transfection, 5×10⁵ Jurkat T cells were plated in round bottom 96-well plates, while 2.5×10⁵ K562 or MSLN overexpressed K562 cells were co-cultured with Jurkat T cells. Meanwhile, a separate medium was set as a negative control, and T cell activator (PMA/Inomycin) was set as a positive stimulation control. The MSLN+ Jurkat T cells were stimulated for 24hrs, and then the cell culture supernatant was collected and centrifuged. Then 10ul supernatant was taken for detection. The activity of secreted luciferase was detected using Gaussia Lucifrease activity detection kit of New England Biolabs company. Compared with the control group that only added with medium, the addition of K562 that did not express MSLN antigen could not stimulate MSLN CAR-positive Jurkat T cells to secrete luciferase, while the addition of K562 cells expressing MSLN antigen could significantly stimulate MSLN CAR-positive Jurkat T cells to secrete luciferase (Figure 6C). The results indicated that after the binding of MSLN CAR-positive Jurkat T cells to MSLN antigen, the transcription factor NFAT was activated and the expression of Gaussia Luciferase, a downstream reporter gene of NFAT was induced in an antigen-specific manner.

At the same time, 293FT cells were transfected with the lentiviral plasmid of Figure 6A. The lentivirus was harvested and T cells isolated from peripheral blood were infected to prepare MSLN CAR positive T cells. The positive T cells infected with MSLN CAR were enriched by immunomagnetic beads and reached a positive rate of 85.8% (Figure 6D). Similarly, K562 cells with high MSLN expression significantly promoted the expression of secreting luciferase, while K562 alone was not stimulating (Figure 6E).

The above two experimental results showed that in the experimental system, MSLN CAR could induce the secretion and expression of downstream genes regulated by NFAT after binding to the specific antigen MSLN.

### 6.2 Construction of MSLN CAR-T cells capable of inducing the secretion of aCD47scFv or aCD47scFv-FC

The operation of 6.1 was repeated, except that the luciferase gene was replaced with aCD47scFv (SEQ ID NO: 2) or aCD47scFV-FC (SEQ ID NO: 4), thereby obtaining T cells comprising the construct shown in Figure 7. The presence of anti-CD47 antibodies in the supernatant was detected using the binding reaction of CD47 antigen and antibody.

The experimental results showed that when MSLN-CAR-T was activated, aCD47scFv or aCD47scFv-FC was detected in the supernatant; while when MSLN-CAR-T was not activated, aCD47scFv or aCD47scFv-FC was almost undetectable in the supernatant. This suggests that in the T cells of the present invention, the CD47 antibody can be efficiently expressed when CAR is activated (or induced activation).

### Example 7 aCD47 scFV secretion in supernatant promotes macrophage phagocytosis

To verify whether the aCD47 scFv single-chain antibody secreted by the transfected cells had a synergistic antitumor effect, the secreted supernatant (containing aCD47 scFv) of lentivirus-infected 293T cells was added to the macrophage/tumor cell co-culture system. Then the phagocytic effects of macrophages on tumors was detected.

Culture of bone marrow-derived macrophage: a femur of C57BL/6 mice is taken and the ends were cut with scissors. The bone marrow in the femur was washed out from one end of the femur by inserting a syringe. Bone marrow-derived cell mixture was collected and centrifuged. Then red blood cell lysate was added to resuspend and remove red blood cells. After washed twice with PBS, the cells were resuspended with macrophage culture medium (10% fetal bovine serum DMEM medium +20% culture supernatant containing M-CSF L929 cells), and spread to a non-adherent cell culture 6-well plate. (1 × 10⁶/well). The cells were cultured for 8 days, and then differentiated into F4/80-staining positive macrophages.

Tumor phagocytosis test: tumor target cells Nalm6 and K562 cells were fluorescently labeled with CFSE (1uM), and then cultured with the pre-cultured bone marrow-derived macrophages in a ratio of 1: 1 (5 × 10⁴: 5×10⁴) in a 96-well plate. After 4 hours of co-culture, the percentage of macrophages that phagocytosed target cells (F4/80+ CFSE+) was analyzed by flow cytometry. In the experimental system of the present invention, 293 cell culture supernatant was used as a control, 100ul of aCD47 scFv secretion culture supernatant was used as a treatment group, and aCD47 antibody (5ug/ml) was used as a positive control group.

The results are shown in Fig. 8. The culture supernatant of 293T cells secreting aCD47 scFv single-chain antibody can promote the phagocytosis of tumor cells Nalm6 (A, B) and K562 (C, D) by bone marrow-derived macrophages.

In addition, the effect of aCD47 scFv-FC was also studied using the same experimental method. It was found that the function and effect of aCD47 scFv-FC on macrophages were almost the same as that of aCD47 scFv.

### Example 8 aCD47scFV promotes synergistic anti-tumor effects of macrophages and MSLN-positive CAR-T

In order to observe whether the MSLN CAR-T cells and macrophages can exert synergistic killing effect on MSLN positive tumor cells in the presence of aCD47scFv from 293T cell, K562 cells stably transfected with firefly luciferase were overexpressed with MSLN antigen, and luciferase was used as an indicator of target cell activity in killing experiments. The luciferase-positive K562 cells were incubated with effector cells (MSLNCAR-T/macrophages) and aCD47scFv supernatant to study the synergistic killing effect of macrophages and CAR-T cells in the presence of aCD47. The experimental design is as follows, MLSN antigen-positive K562 target cells were subjected to different experimental treatments respectively. The treatments were as follows:
Experimental group 1. 293T cell culture supernatant (control group)
Experimental group 2. Macrophages + 293T cell culture supernatant
Experimental group 3. Macrophages + aCD47scFv cell supernatant
Experimental group 4. MSLN CAR-T cells + 293T cell culture supernatant
Experimental group 5. MSLN CAR-T cells + aCD47scFv cell supernatant
Experimental group 6. MSLN CAR-T cells + macrophages + cell culture supernatant
Experimental group 7. MSLN CAR-T cells + macrophages + aCD47 scFv cell supernatant

After co-culture for 5 hours, the culture was centrifuged, the luciferase substrate was added, and the number of viable cells was measured. It was found that in the presence of aCD47 scFV cell supernatant, macrophages could synergistically promote the killing of MSLN-positive CAR-T cells to target cells, MSLN-antigen-positive K562 cells (Figure 9).

In addition, the present invention also studied the effect of aCD47 scFv-FC. The experimental method is the same as aCD47 scFV, wherein aCD47 scFv is replaced with aCD47 scFv-FC. It was found that the function and effect of aCD47 scFv-FC were almost the same as aCD47 scFv.

In the present invention, MSLN CAR-T cells capable of inducing secretion of aCD47scFv or aCD47scFv-FC prepared in Example 6 and macrophage cells (Experimental Group 8) were co-cultured with the above luciferase-positive K562 cells. The experimental method is the same as above.

It was found that the results of experimental group 8 were almost the same as those of experimental group 7, wherein the luciferase activity was slightly lower than that of experimental group 7.

The results showed that the killing effect of experimental groups 7 and 8 on target cell, MSLN antigen-positive K562 cells was significantly stronger than that of other experimental groups, and the killing effect of experimental group 8 was the strongest. It shows that anti-CD47 antibodies (such as aCD47 scFv and aCD47scFv-FC) and CAR targeting MSLN have a synergistic effect and can kill tumor cells more effectively. Moreover, the CAR-T cells of the present invention induce the secretion of an anti-CD47 antibody when the CAR targeting MSLN is activated. Therefore, the CAR-T cells are safer and have less toxic and side effects.

### Example 9

The experimental method was the same as that of Examples 6, 7 and 8. CD19 CAR-T cells capable of inducing the secretion of aCD47scFv were prepared, and their killing effect on target cells was verified.

The results show that after the CAR of CD19 CAR-T cells capable of inducing the secretion of aCD47scFv binds to the antigen, aCD47scFv or aCD47scFv-FC can be effectively expressed and tumor cells can be more effectively killed with less toxic and side effects and it is safer.

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

## Claims

1. An engineered immune cell, wherein the engineered immune cell is a T cell or an NK cell with following characteristics:
(a) the immune cell expresses a chimeric antigen receptor CAR or an exogenous TCR, wherein the CAR targets a marker of tumor cells, and the exogenous TCR targets a marker of tumor cells; and
(b) when the CAR is activated and/or the exogenous TCR is activated, the immune cell induces the secretion of anti-CD47 antibodies.

2. The immune cell of claim 1, wherein the engineered immune cell is selected from the group consisting of:
(i) chimeric antigen receptor T cell (CAR-T cell);
(ii) chimeric antigen receptor NK cell (CAR-NK cell); or
(iii) exogenous T cell receptor (TCR) T cell (TCR-T cell).

3. The immune cell of claim 1, wherein the structure of the CAR is shown in formula I:
L1-scFv-H1-TM-C-CD3ζ (I)
wherein,
L1 is none or a signal peptide sequence;
scFv is an antigen binding domain;
H1 is none or a hinge region;
TM is a transmembrane domain;
C is a co-stimulatory signaling molecule;
CD3ζ is a cytoplasmic signaling sequence derived from CD3ζ;
the "-" is a linker peptide or a peptide bond;

4. The immune cell of claim 1, wherein the anti-CD47 antibody is an anti-CD47 scFv, and the structure of the anti-CD47 scFv is shown in formula II as below:
L2-VH-X-VL-H2-G (II)
wherein,
L2 is none or a signal peptide sequence;
VH is a heavy chain variable region of anti-CD47 antibody;
X is none or a linker peptide;
VL is a light chain variable region of anti-CD47 antibody;
H2 is none or a hinge region of an immunoglobulin;
G is none or an Fc fragment.

5. The immune cell of claim 1, wherein the anti-CD47 antibody is selected from the group consisting of an animal-derived antibody, a chimeric antibody, a humanized antibody, and a combination thereof.

6. The immune cell of claim 1, wherein the anti-CD47 antibody is a partially or fully humanized antibody.

7. The immune cell of claim 1, wherein the anti-CD47 antibody is in a form of single-chain or double-chain.

8. The immune cell of claim 4, wherein amino acid sequence of the anti-CD47 scFv is as shown in SEQ ID NO: 2, SEQ ID NO: 4 or SEQ ID NO: 6.

9. A method for preparing the immune cell of claim 1, comprising the following steps:
(A) providing a immune cell to be modified; and
(B) modifying the immune cell to express CAR or the exogenous TCR, wherein when the CAR is activated and/or the exogenous TCR is activated, the immune cell induces the secretion of anti-CD47 antibodies, thereby obtaining the engineered immune cell of claim 1.

10. The method of claim 9, wherein the step (B) comprises (B1) transferring a first expression cassette expressing the CAR or exogenous TCR into the immune cell; and (B2) transferring a second expression cassette which can induce the secretion of anti-CD47 antibodies into the immune cell; and the step (B1) may be performed before, after, at the same time, or alternately with step (B2);
wherein the second expression cassette has a structure of formula III from 5'-3':
Z1-Z2 (III)
wherein,
each "-" is independently a bond or a nucleotide linking sequence;
Z1 is an inducible promoter;
Z2 is a nucleic acid sequence encoding an anti-CD47 antibody.

11. A preparation comprising the immune cell of claim 1, and a pharmaceutically acceptable carrier, diluent or excipient.

12. A use of the engineered immune cell of claim 1 for the preparation of a medicament or a preparation for preventing and/or treating cancer or tumor.

13. A kit for preparing the engineered immune cell of claim 1 including a container and following components located in the container:
(1) a first nucleic acid sequence comprising a first expression cassette for expressing the CAR or exogenous TCR; and
(2) a second nucleic acid sequence comprising a second expression cassette for inducing secretion of anti-CD47 antibodies.
